# EUROPEAN PATENT APPLICATION

(11) **EP 4 640 108 A1**
(43) Date of publication of application: **29.10.2025**
(21) Application number: 24171672.9
(22) Date of filing: 22.04.2024
(51) Int. Cl.: A45D 34/00, A45D 40/00, A61K 8/06

(54) **SUSTAINABLE PRODUCT**

(71) Applicant: Beiersdorf AG, 22529 Hamburg (DE)
(72) Inventor: Janz, Adriana Christina, 22159 Hamburg (DE); Timm, Sebastian, 22765 Hamburg (DE); Franck, Kerstin, 25451 Quickborn (DE); Rinaldi, Estée Marie Lou, 20225 Hamburg (DE); Wehmann, Martin, 24226 Heikendorf (DE); Sartori, Antonio Augusto, 22525 Hamburg (DE)
(74) Representative: Beiersdorf AG

(57) **Abstract**

The present invention belongs to the cosmetic field.

## Description

The present invention belongs to the cosmetic field and relates to a cosmetic product including a cosmetic emulsion.

A beautiful and attractive appearance is a desire for many people. One typical sign of such an appearance is a healthy and smooth looking skin. In order to take care on the skin, it is for many people a daily routine to apply cosmetic products such as emulsions, in particular body or face care products.

Products such as face care products are conveniently sold in plastic container having a volume suitable for a filling of approximately 20 to 600 ml. The plastic container is formed using synthetic polymers. Most of the materials used to produce polymers for plastic container applications, such as polyethylene, polyethylene terephthalate, and polypropylene, are derived from monomers (e.g., ethylene, propylene, terephthalic acid, ethylene glycol), which are obtained from non-renewable, fossil-based resources, such as petroleum, natural gas, and coal. Thus, the price and availability of the petroleum, natural gas, and coal feedstock ultimately have a significant impact on the price of polymers used for plastic container materials. As the worldwide price of petroleum, natural gas, and/or coal escalates, so does the price of plastic container materials. Furthermore, many consumers display an aversion to purchasing products that are derived from petrochemicals. In some instances, consumers are hesitant to purchase products made from limited non-renewable resources (e.g., petroleum, natural gas and coal). Other consumers may have adverse perceptions about products derived from petrochemicals as being "unnatural" or not environmentally friendly.

It has been noted that the packaging material may significantly affect the smell of the head space of cosmetic products, if stored for 4 months at 40°C or 6°C. In some cases, the smell of the head space of cosmetic products may even be affected if stored for 4 months under room temperature, or if the composition is exposed to UV radiation for 4 months in a transparent packaging. For the case a consumer wants to test such a product in a shop the consumer often opens the lid or cap of the packaging and smells on the product. In this way consumers often evaluate the perfume of the product. However, in the cases described above the smell of of the composition may have deteriorated.

An environmental alternative to virgin plastic is plastic comprising post-consumer recycled resin (PCR). PCR is preferably made from the mechanical recycling of plastic, typically packaging plastic and is described in Mechanical Recycling of Packaging Plastics: A Review, Z. O. G. Schyns; M. P. Shaver Macromol. Rapid. Common. 2021, 42, 2000415.

The PCR may comprise any of the plastics typically used in providing raw material for containers for cosmetic products, for example polyethylene (PE), polypropylene (PP), and polyethylene terephthalate (PET).

As such it is a desire to provide consumers with environmentally friendly packaged products with no deteriorated smell.

Although PCR is an environmentally friendly alternative to virgin plastics for packaging of cosmetic applications, in general, PCR has sometimes malodor originating from volatile organic compounds coming from previous fillings and thermal treatment during the mechanical recycling process.

It was now surprisingly found by the applicant that the object of the present invention allows to reduce the malodors occurring in specific cosmetic emulsion and packaging under the conditions described above.

The object of the present invention is a cosmetic product comprising
a) A container which material comprises at least 50% by weight of the total material of the container of post-consumer recycled polypropylene and/or polyethylene, and
b) A cosmetic emulsion, which is characterized in that it is a water in oil emulsion,
wherein the emulsion is located inside of the container.

It was found that the cosmetic emulsion and the packaging were capable to avoid the deterioration which might occur upon storage at the above outlined conditions.

The document WO 2012/102778 A1 discloses cosmetic container comprising recycled and bio-based plastic. However, there is disclosure how malodors caused by post-consumer recycled plastic can be addressed.

US 5350788A discloses a method of reducing malodors caused by recycled polymeric material such as plastic, which includes the step of incorporating a polyalkylene imine into the polymeric plastic material. However, the disadvantage of this method is that production cost of container made of post-consumer recycled plastic may be increased, due to a chemical modification of the plastic mixture.

There is no disclosure that malodors may not occur in PCR if a water in oil emulsion is employed.

All the weight percentages (% by weight) given below relate, unless otherwise stated, to the total weight of the cosmetic emulsion.

If ratios of certain components are disclosed in the following description, these ratios refer, unless otherwise stated, to weight ratios of the components.

Unless otherwise stated, all tests and measurements were performed under "normal conditions". The term "normal conditions" refers to 20°C, 1013 hPa and a relative humidity of 50%.

In the following description the terms "according to the invention", "preferred according to the invention" and so on are always directed to the use according to the invention, to the composition and to the method according to the invention.

For the purposes of the present disclosure, the term "free from" means that the proportion of the respective substance is less than 0.01% by weight. This ensures that entrainments or impurities with these substances are not included as "free from" according to the invention.

The term "skin" refers solely to the human skin.

Emulsifiers are understood to be all substances which are listed in the International Cosmetic Ingredient Dictionary and Handbook, Thirteenth Edition 2010, (ISBN 1-882621-47-6) under the name "emulsifying agent". Surfactants are understood to be all substances which are listed in the International Cosmetic Ingredient Dictionary and Handbook, Thirteenth Edition 2010, (ISBN 1-882621-47-6) under the name "surfactant".

The term "a container comprising at least 50% by weight of post-consumer recycled plastic" is understood as the material the container is composed of comprises at least 50% by weight of post-consumer recycled plastic. For the cases the container is described, the term "comprising" is always used to describe the material of which the container is made of. Similar descriptions must be interpreted in analog manner.

Preferably, the container comprises of at least 30%, more preferably at least 65%, more preferably at least 75% by weight PCR polypropylene, based on the total weight of the container.

Further in some embodiments of the invention the container comprises of at least 30%, more preferably at least 65%, more preferably at least 75% by weight PCR polyethylene, based on the total weight of the container.

It is most preferred if the container either comprises of at least 99% by weigh of PCR polypropylene or at least 99% by weight PCT polyethylene, based on the total weight of the container.

Further the container may comprise further plastic material which is selected from PE, PET and mixtures thereof. It is preferred if those plastics are also obtained from /are made of PCR.

In some examples it is preferred if the PE is characterized in that it contains HDPE, LLDPE and/or LDPE.

In one aspect of the invention the container is characterized in that it comprises multiple layers of plastic in respect of the thickness of the container wall. The layers may be made of different kinds of plastics. Preferred plastic materials are outlined above.

According to the invention it is preferred if the container has a inner filling volume of 10 to 600 ml, preferably 20 to 400 ml and most preferably 30 to 260 ml.

The container is preferably injection or blow moulded. Blow moulding involves the formation of a parison or preform which is placed and clamped into the mould. Air is passed into the parison/preform to expand the parison/preform such that it expands to fill the space in the mould. Once the plastic has hardened sufficiently, the mould is de-coupled, and the moulded article is removed. Injection moulding involves injection of molten PCR into a mold cavity which compromises the shape of the packaging intended for the application under elevated temperature and pressure. The molten PCR cools and solidifies in the mold cavity and the solidified packaging article is ejected from the mold cavity.

Preferably container comprises at least one colourant masterbatch. For the case that the container is build up by at least two plastic layers, it is preferred if the outer and/or inner layer comprises a colourant masterbatch. More preferably, the outer layer comprises a colourant masterbatch. By "colourant masterbatch" it is a meant a mixture in which pigments are dispersed at high concentration in a carrier plastic material. The colorant masterbatch is used to impart colour to the article. The carrier may be a biobased plastic or a petroleum-based plastic, or a biobased oil or a petroleum-based oil or made of post-consumer resin (PCR). The pigment of the carrier may include, for example, an inorganic pigment, an organic pigment, a polymeric resin, or a mixture thereof.

Optionally, the colourant masterbatch can further include one or more additives. Nonlimiting examples of additives include slip agents, UV absorbers, nucleating agents, UV stabilizers, heat stabilizers, clarifying agents, fillers, brighteners, process aids, perfumes, flavors, and a mixture thereof.

It is advantageous according to the invention if the amount of the water phase is 35 to 75% by weight and the amount of the oil phase is 25 to 65% by weight, based on the total amount of the emulsion (emulsion).

It is preferred according to the invention if the emulsion is free from aliphatic and aromatic hydrocarbons.

Embodiments of the present invention that are advantageous according to the invention are characterized in that the emulsion additionally contains straight-chain and/or branched fatty alcohols.

It is preferred according to the invention if octyldodecanol, cetyl alcohol and/or stearyl alcohol are used as straight-chain and/or branched fatty alcohols. It is also preferred according to the invention to use these straight-chain and/or branched fatty alcohols in a total concentration of 0.3 to 2.5% by weight, based on the total weight of the emulsion.

Embodiments of the present invention that are advantageous according to the invention are characterized in that the emulsion is emulsified with the emulsifier diisostearoyl polyglyceryl-3 dimer dilinoleate. This can be purchased, for example, under the trade name Isolan PDI from Evonik. It is preferred according to the invention if the emulsion contains diisostearoyl polyglyceryl-3 dimer dilinoleate in a concentration of 1.5 to 2.5% by weight, based on the total weight of the emulsion.

According to the invention it is preferred if the composition comprises at least one triglyceride. It is advantageous according to the invention if the content of triglycerides in the emulsion is from 10 to 30% by weight, based on the total weight of the emulsion. According to the invention the preferred concentration range is from 20 to 27% by weight, based on the total weight of the emulsion.

Advantageous embodiments of the present invention are further characterized in that the emulsion contains lecithin and/or glycerin. The preferred use concentration for lecithin according to the invention is 0.0005 to 0.1% by weight, based on the total weight of the emulsion.

The use concentration for glycerol which is preferred according to the invention is 2 to 6% by weight, based on the total weight of the emulsion.

In addition, it is advantageous according to the invention if the emulsion according to the invention contains one or more esters selected from the group consisting of the compounds ascorbyl palmitate, tocopheryl acetate and cetyl palmitate.

The preferred use concentration according to the invention for ascobyl palmitate is 0.0003 to 0.1% by weight, based on the total weight of the emulsion.

The preferred use concentration according to the invention for tocopheryl acetate is 0.0001 to 0.1% by weight, based on the total weight of the emulsion.

The use concentration for cetyl palmitate which is preferred according to the invention is 0.005 to 1.0% by weight, based on the total weight of the emulsion.

It is advantageous according to the invention if the emulsion contains one or more salts selected from the group consisting of the compounds magnesium sulfate, sodium anisate and sodium citrate. Sodium citrate, which can also be used in the form of its free acid (citric acid), can be used here, which is preferred according to the invention.

The concentration of magnesium sulfate that is preferred according to the invention is 0.3 to 1.5% by weight, based on the total weight of the emulsion.

The preferred use concentration according to the invention for sodium anisate is 0.002 to 0.9% by weight, based on the total weight of the emulsion.

The concentration of citric acid or sodium citrate that is preferred according to the invention is 0.001 to 0.8% by weight, based on the total weight of the emulsion.

Embodiments of the present invention that are advantageous according to the invention are characterized in that the emulsion contains phenoxyethanol and/or panthenol.

The use concentration for phenoxyethanol which is preferred according to the invention is 0.01 to 0.7% by weight, based on the total weight of the emulsion.

The preferred use concentration for panthenol according to the invention is 0.05 to 0.94% by weight, based on the total weight of the emulsion.

According to the invention, the use of a combination of panthenol, in particular D-panthenol, and glycerol is particularly preferred. The advantageous embodiments of the present invention according to the invention are characterized in that the triglycerides are selected from mixtures of hydrogenated rapeseed oil, rapeseed oil, caprylic/capric acid triglycerides, sunflower oil, hydrogenated coconut glycerides, hydrogenated castor oil and shea oil, the mixture preferably consisting of several or all of these triglyceride sources exists.

If the emulsion according to the invention contains hydrogenated rapeseed oil (INCI: Hydrogenated Rapeseed Oil), this is preferably used according to the invention in a concentration of 4 to 15% by weight, based on the total weight of the emulsion.

If the emulsion according to the invention contains caprylic/capric acid triglycerides (INCI: Caprylic/Capric Triglycerides), this is preferably used according to the invention in a concentration of 5.1 to 10% by weight, based on the total weight of the emulsion.

If the emulsion according to the invention contains rapeseed oil (INCI: Brassica Campestris Seed Oil), this is preferably used according to the invention in a concentration of 2 to 4% by weight, based on the total weight of the emulsion. If the emulsion according to the invention contains sunflower oil (in particular that with the INCI Heliathus Annuus Hybrid Oil), this is preferably used according to the invention in a concentration of 1.6 to 5% by weight, based on the total weight of the emulsion.

### Examples

The following examples are intended to illustrate the present invention without restricting it. Unless otherwise stated, all amounts, parts and percentages are based on the weight and the total amount or the total weight of the emulsions.

| Ingrediens (INCI) | Ex. 1 | Ex.2 | Ex.3 | Ex.4 |
|---|---|---|---|---|
| Aqua | Ad. 100 | Ad. 100 | Ad. 100 | Ad. 100 |
| Hydrogenated Rapeseed Oil | 8.47 | 6.12 | 4.15 | 7.10 |
| Brassica Campestris Seed Oil | 2.55 | 2.28 | 3.22 | 3.32 |
| Hydrogenated Coco Glycerides | 1.64 | 1.53 | 1.85 | 1.27 |
| Butyrospermum Parkii Oil | 1.43 | 1.94 | 1.75 | 1.79 |
| Diisostearoyl Polyglyceryl-3 Dimer Dilinoleate | 1.60 | 1.80 | 2.20 | 2.00 |
| Caprylic/Capric Triglycerides | 5.50 | 9.50 | 7.00 | 5.70 |
| Helianthus Annuus Hybrid Oil | 4.00 | 2.00 | 4.80 | 4.75 |
| Hydrogenated Castor Oil | 0.40 | 0.40 | 0.40 | 0.40 |
| Cetearyl Alcohol | 0.33 | 0.70 | 0.50 | 0.30 |
| Octyldodecanol | 0.66 | 0.80 | 1.00 | 0.75 |
| Cetyl Palmitate | 0.90 | 0.50 | 0.70 | 0.10 |
| Lecithin | 0.0055 | 0.0073 | 0.0033 | 0.0068 |
| Ascorbyl Palmiate | 0.0045 | 0.0033 | 0.0077 | 0.0041 |
| Parfum | 0.17 | 0.20 | 0.22 | 0.15 |
| Glycerin | 3.00 | 4.50 | 5.50 | 2.50 |
| Panthenol | 0.80 | 0.50 | 0.40 | 0.20 |
| Magnesium Sulfate | 0.50 | 0.60 | 0.80 | 0.70 |
| Phenoxyethanol | 0.20 | 0.15 | 0.10 | 0.02 |
| Pantolactone | 0.0213 | 0.013 | 0.0106 | 0.005 |
| Sodium Anisate | 0.10 | 0.15 | 0.20 | 0.06 |
| Citric Acid | 0.04 | 0.06 | 0.08 | 0.02 |

The emulsions above are characterized by that they are water in oil emulsions. The emulsions are prepared such as conventional water in oil emulsion, as known by a person skilled in art.

Emulsions of the disclosed type are put into different container. The first container to be used was one made of 100% by weight of virgin PP. The second container to be used was made of 100% by weight of different PCR grades from different suppliers.

In one example, a white sorted PCR grade which has density of 0,919 g/cm³ (ISO 1183), melt flow rate of 19 g/10 min (ISO 1133 - 230 °C/2.16 kg) and notched Charpy Impact Strength (notched) 4,9 kJ/m² (ISO 179 - 23°C) and tensile modulus of 1440 MPa (ISO 527 - 23°C) is used.

In another example, a grey sorted PCR grade which has a melt flow rate of 17 g/10 min (ISO 1133 - 230 °C/2.16 kg) and tensile modulus of 1440 MPa (ISO 527- 23°C) is used.

In another example, a white sorted PCR grade which has a density of 0,915 g/cm³ (ISO 1183), melt flow rate of 19 g/10 min (ISO 1133 - 230 °C/2.16 kg) and notched Charpy Impact Strength (notched) 6,5 kJ/m² (ISO 179 - 23°C), tensile modulus of 1250 MPa (ISO 527-2 - 23°C) and elongation at break of 30% (ISO 527-2 - 23°C) is used.

In another example, a white sorted PCR grade which has a density of 0,92 g/cm³ (ISO 1183), melt flow rate of 11,5 g/10 min (ISO 1133 - 230 °C/2.16 kg) and notched Charpy Impact Strength (notched) 5,8 kJ/m² (ISO 179 - 23°C), tensile modulus of 1100 MPa (ISO 527-2 - 23°C) and elongation at break of 20% (ISO 527-2 - 23°C) is used.

The PCR grades mentioned here are degassed / deodorized after extrusion process to remove the volatile organic compounds by purging hot air for prolonged times.

The containers were sealed, and each type exposed to different conditions.

The first condition to be tested was storing at 6°C for 4 months. Further, a test was performed at 40°C for 4 months. A further test was performed at room temperature for the same period of time.

After exposure the containers were opened, and the head space smell was analyzed by an expert panel. Further a smell card analysis was performed. For that purpose, a panel of 9 panelist was used to evaluate the smell of the head space and on a smell card. A score was given based on the evaluation. The sore was on a scale from 1.0 to 3.0 and the score war related to the same composition packaged in virgin plastic and stored for 6°C. A score of 3.0 corresponded to a good smell while 1.0 corresponded to a bad smell.

A similar test was performed using a conventional Nivea Soft Cream, which is an oil in water emulsion. The INCI of this formulations contains: Aqua, Glycerin, Myristyl Alcohol, Alcohol Denat., Myristyl Myristate, Glyceryl Stearate, Coco-Caprylate/Caprate, Palmitic Acid, Stearic Acid, Cetearyl Glucoside, Hydrogenated Coco-Glycerides, Simmondsia Chinensis Seed Oil, Tocopheryl Acetate, Prunus Amygdalus Dulcis Oil, Vegetable Oil, Cetyl Ricinoleate, Triisostearin, Sorbitan Stearate, Hydroxypropyl Starch Phosphate, Sodium Hydroxide, Cetearyl Alcohol, Gellan Gum, Xanthan Gum, Myristic Acid, Arachidic Acid, Oleic Acid, Disodium Cetearyl Sulfosuccinate, Phenoxyethanol, Linalool, Citronellol, Alpha-Isomethyl Ionone, Limonene, Parfum.

However, for the oil in water emulsion it was found that the head space analysis resulted in a slightly worse head space smell after 4 months exposure at 40°C for a container made of PCR.

As such it can be stated that water in oil emulsion maintain a better head space and smell score if stored in PCR PP at 6°C or 40°C. Other formulations instead show a worsening effect. Thus, environmentally friendly products can be provided without affecting the properties of the product.

## Claims

1. Cosmetic product comprising
a) A container which material comprises at least 50% by weight of the total material of the container of post-consumer recycled polypropylene and/or polyethylene, and
b) A cosmetic emulsion, which is **characterized in that** it is a water in oil emulsion,
wherein the preparation is located inside of the container.

2. Product according to claim 1 **characterized in that** the container comprises of at least 30%, more preferably at least 65%, more preferably at least 75% by weight PCR polypropylene, based on the total weight of the container.

3. Product according to claim 1 **characterized in that** the container comprises of at least 30%, more preferably at least 65%, more preferably at least 75% by weight PCR polyethylene, based on the total weight of the container.

4. Product according to any of the proceeding claims **characterized in that** the container either comprises of at least 99% by weigh of PCR polypropylene or at least 99% by weight PCT polyethylene, based on the total weight of the container.

5. Product according to claim 1, 3 or 4 **characterized in that** the polyethylene is **characterized in that** it contains HDPE, LLDPE and/or LDPE.

6. Product according to any of the proceeding claims **characterized in that** the container has an inner filling volume of 10 to 600 ml, preferably 20 to 400 ml and most preferably 30 to 260 ml.

7. Product according to any of the proceeding claims **characterized in that** the container is injection or blow moulded.

8. Product according to any of the proceeding claims **characterized in that** the container comprises at least one colourant masterbatch.

9. Product according to any of the proceeding claims **characterized in that** the amount of the water phase is 35 to 75% by weight and the amount of the oil phase is 25 to 65% by weight in the emulsion, based on the total amount of the emulsion.

10. Product according to any of the proceeding claims **characterized in that** the content of triglycerides in the emulsion is from 10 to 30% by weight, based on the total weight of the emulsion.

11. Product according to any of the proceeding claims **characterized in that** the emulsion additionally contains straight-chain and/or branched fatty alcohols.

12. Product according to any of the proceeding claims **characterized in that** the emulsion is free from aliphatic and aromatic hydrocarbons.

13. Product according to any of the proceeding claims **characterized in that** the emulsion comprises glycerol in a quantity in the range from 2 to 6% by weight, based on the total weight of the emulsion.
